(19) Europäisches Patentamt
European Patent Office
Office européen des brevets

(11) Numéro de publication: **0 263 020 B1**

(12) # FASCICULE DE BREVET EUROPEEN

(45) Date de publication du fascicule du brevet: **25.07.90**

(51) Int. Cl.⁵: **C07D 277/42**, A61K 31/425

(21) Numéro de dépôt: **87402120.7**

(22) Date de dépôt: **23.09.87**

(54) **Dérivés de 4-phénylthiazole.**

(30) Priorité: **02.10.86 FR 8613739**

(43) Date de publication de la demande: **06.04.88 Bulletin 88/14**

(45) Mention de la délivrance du brevet: **25.07.90 Bulletin 90/30**

(84) Etats contractants désignés: **AT BE CH DE ES FR GB GR IT LI LU NL SE**

(56) Documents cités:
**CHEMICAL ABSTRACTS, vol. 94, no. 23, 8 juin 1981, page 657, résumé no. 192274c, Columbus, Ohio, US; R. RASTOGI et al.: "Synthesis of N-(2-substituted-ethyl)-N'-arylpiperazines and their quaternary salts as potential antihookworm agents", & INDIAN. J. CHEM. SECT. B 1980, 19B(11), 1003-513, 33-42hloric acid to remove scale from heat exchangers", & K 000**

(73) Titulaire: **LABORATOIRE L. LAFON Société anonyme dite:, 1 rue Georges Médéric, F-94701 Maisons-Alfort(FR)**

(72) Inventeur: **Lafon, Louis, 5, rue de l'Alboni, F-75016 Paris(FR)**

(74) Mandataire: **Clisci, Serge et al, S.A. FEDIT-LORIOT CONSEILS EN PROPRIETE INDUSTRIELLE 38, avenue Hoche, F-75008 Paris(FR)**

EP 0 263 020 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne l'utilisation thérapeutique de dérivés de 4-phénylthiazole, à savoir le 2-(2-hydroxyéthylamino)-4-phénylthiazole et ses sels d'addition. Elle crise plus particulièrement leur procédé de préparation et leur utilisation en thérapeutique, notamment en tant qu'agents sédatifs.

On connaît le 2-(2-hydroxyéthylamino)-4-phénylthiazole en tant que curiosité de laboratoire de l'article de Rastogi et al., Indian J. Chem., Sect. B 19B (11), pages 1003–1005, (1980) tel que résumé dans CA 94, 192 274c.

Suivant l'invention on préconise une utilisation thérapeutique de ce produit et de ses sels d'addition, notamment en tant qu'agents sédatifs. Les dérivés de 4-phénylthiazole suivant l'invention dont on préconise l'utilisation en thérapeutique caractérisés en ce qu'ils sont choisis parmi l'ensemble comprenant

a) le 2-(2-hydroxyéthylamino)-4-phénylthiazole de formule développée:

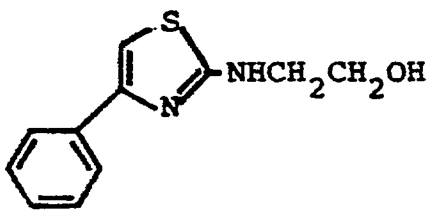

(I)

et,

b) ses sels d'addition.

Par sels d'addition, on entend ici, d'une part, les sels d'addition d'acide obtenus par réaction de la base libre de formule I avec les acides minéraux et organiques et, d'autre part, les sels d'ammonium. Parmi les acides utilisables pour sailifier la base de formule I, on peut notamment mentionner les acides chlorhydrique, bromhydrique, acétique, formique, propionique, oxalique, fumarique, maléique, succinique, benzoïque, cinnamique, mandélique, citrique, malique, tartrique, aspartique, glutamique, méthanesulfonique, p-toluènesulfonique. Parmi les composés permettant d'obtenir des sels d'ammonium, on peut notamment citer $ICH_3$, $ClCH_3$, $ICH_2C_6H_5$ et $ClCH_2C_6H_5$. Les sels d'addition d'acide sont les sels préférés d'un point de vue thérapeutique; parmi lesdits sels d'addition d'acide, le chlorhydrate est l'un des plus intéressants en tant qu'agent sédatif.

Un certain nombre de composés typiques suivant l'invention a été consigné dans le tableau I ci-après.

TABLEAU I

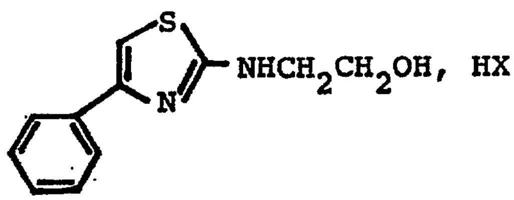

| Produit | No. de code | HX |
|---|---|---|
| Ex. 1 | CRL 41108 | HCl |
| Ex. 2 | - | $CH_3SO_3H$ |
| Ex. 3 | - | 1/2 acide fumarique |
| Ex. 4 | - | 1/2 acide maléique |
| Ex. 5 (a) | - | - |

Note
(a) : base libre

Le 2-(2-hydroxyéthylamino)-4-phénylthiazole et ses sels d'addition peuvent être préparés selon une méthode connue en soi par application de mécanismes réactionnels classiques. Le procédé que l'on préconise selon l'invention consiste à remplacer l'un des atomes d'hydrogène du groupe 2-amino du 2-amino-4-phénylthiazole par le groupe β-hydroxyéthyle.

Le meilleur mode de mise en oeuvre de ce procédé, qui est illustré par le schéma A :

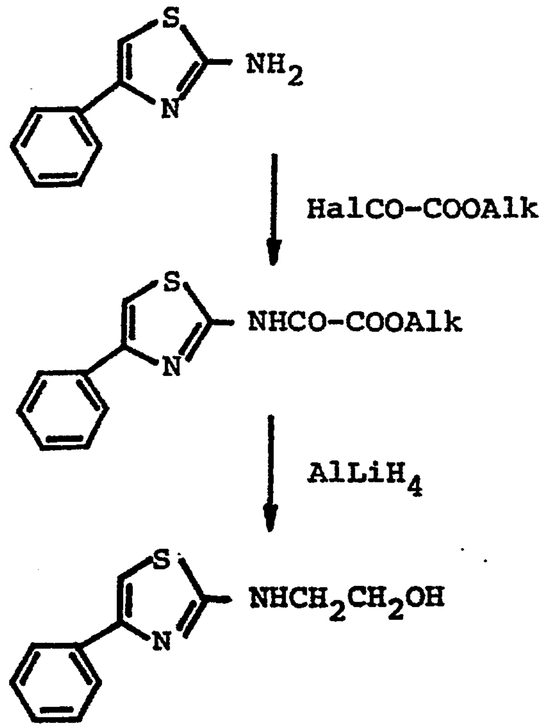


(II)

(III)

(IV)

(I)

(où Alk représente un groupe alkyle inférieur, notamment en $C_1$-$C_2$, et de préférence le groupe $CH_2CH_3$; et, Hal représente un atome d'halogène, notamment F, Cl,Br, l'halogène préféré étant le chlore), est caractérisé en ce que

1°) on fait réagir le 2-amino-4-phénylthiazole (II) avec un halogénure d'un monoester d'alkyle inférieur de l'acide oxalique (III), pour obtenir un 2-alkyloxalylamido-4-phénylthiazole (IV), puis

2°) on réduit les fonctions amide et ester dudit 2-alkyloxalylamido-4-phénylthiazole (IV), ainsi obtenu, au moyen de $AlLiH_4$.

La réaction II + III → IV du stade 1°) est avantageusement mise en oeuvre dans un solvant organique anhydre pendant au moins 0,25 h, à raison de plus d'une mole de III pour une mole de II (en particulier 1,1 mole de III pour 1 mole de II), en présence d'un moyen accepteur de proton (notamment une base organique telle que la pyridine et les $\alpha$, $\beta$, et $\gamma$-picolines), ladite base organique pouvant intervenir en tant que solvant ou co-solvant de ladite réaction.

La réaction IV + $AlLiH_4$ → I du stade 2°) est réalisée dans un solvant organique anhydre (de préférence le tétrahydrofuranne) à raison de plus de deux moles de $AlLiH_4$ pour une mole de IV (en particulier 3 moles de $AlLiH_4$ pour 1 mole de IV).

Le 2-amino-4-phénylthiazole utilisé comme matière première dans ce procédé peut être préparé selon une méthode connue ou banale en soi, par exemple selon le résumé des Chemical Abstracts 60, 4123c ou la Préparation II ci-après.

On a constaté que le 2-(2-hydroxyéthylamino)-4-phénylthiazole et ses sels d'addition sont des agents psychotropes ; ils agissent sur le système nerveux central (SNC) en tant que moyens sédatifs. Selon l'invention on préconise une composition thérapeutique, qui est caractérisée en ce qu'elle renferme, en association avec un excipient physiologiquement acceptable, au moins un composé choisi parmi le 2-(2-hydroxyéthylamino)-4-phénylthiazole et ses sels d'addition non-toxiques.

Bien entendu dans une telle composition, le principe actif, à savoir ici le 2-(2-hydroxyéthylamino)-4-phénylthiazole, ses sels d'addition non-toxiques et leurs mélanges, intervient en quantité pharmaceutiquement efficace.

Selon l'invention on préconise également l'utilisation d'une substance appartenant à l'ensemble comprenant le 2-(2-hydroxyéthylamino)-4-phénylthiazole et ses sels d'addition non-toxiques, pour l'obtention d'un médicament sédatif destiné à une utilisation en thérapeutique vis-à-vis des maladies et pathologies exigeant un sédatif pour calmer et pour tranquilliser les patients.

D'autres avantages et caractéristiques de l'invention seront mieux compris à la lecture qui va suivre d'exemples de préparation et de résultats d'essais neuropsychopharmacologiques, l'ensemble de ces éléments n'étant pas limitatif mais donné à titre d'illustration.

PREPARATION I

Obtention du chlorhydrate de 2-(2-hydroxyéthylamino)-4-phénylthiazole

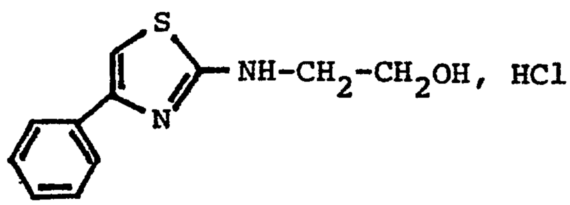

(Exemple 1; No. de code : CRL 41 108)

a) 2-(Ethyloxalylamido)-4-phénylthiazole

Au sein d'une solution de 17,6 g (0,10 mole) de 2-amino-4-phénylthiazole dans 25 ml de pyridine, on coule en 0,5 h 12,3 ml (0,11 mole) de chlorure d'éthyloxalyle ($ClCOCOOC_2H_5$; autres nomenclatures : chloroformylformiate d'éthyle, chlorooxoacétate d'éthyle). On dilue avec 100 ml d'acétone, agite pendant 0,5 h et verse la suspension résultante dans de l'eau froide. Il se forme un précipité que l'on recueille par filtration. Par recristallisation de l'éthanol anhydre on obtient 24,8 g (rendement : 89,85%) de 2-(éthyloxalylamido)-4-phénylthiazole de formule

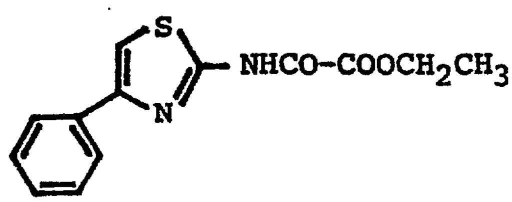

se présentant sous la forme d'une poudre jaune. $F_{inst.}$ = 159°C.

b) CRL 41 108

Au sein d'une suspension de 6,85 g (0,18 mole) de $AlLiH_4$ dans 200 ml de tétrahydrofuranne, on coule en 1,5 h une solution de 16,56 g (0,06 mole) de 2-(éthyloxalylamido)-4-phénylthiazole [obtenu comme indiqué au stade a) ci-dessus] dans 200 ml de tétrahydrofuranne. On poursuit l'agitation du mélange résultant pendant 1,5 h puis neutralise l'excès de $AlLiH_4$ par 36 ml d'acétate d'éthyle et décompose les complexes ainsi obtenus par additions successives de 6,9 ml d'eau, 6,9 ml de soude 4N et 21 ml d'eau. On élimine l'insoluble par filtration, sèche le filtrat sur sulfate de sodium sec et évapore à sec sous pression réduite ledit filtrat ainsi séché. Le résidu d'évaporation résultant [qui contient le 2-(2-hydroxyéthylamino)-4-phénylthiazole] est traité par de l'éthanol chlorhydrique dans l'éther diéthylique et le précipité formé est purifié par 2 recristallisations successives, avec traitement au noir CXA, de l'isopropanol et de l'acétonitrile, pour donner 9,5 g (rendement : 61,7%; rendement global a) + b) : 55,4%) de CRL 41 108 sous la forme d'une poudre blanche soluble dans l'eau. F = 75°C environ.

PREPARATION II

Obtention du 2-amino-phénylthiazole
(No. de code : CRL 41 081)

On porte au reflux pendant 2 h une solution de 19,9 g (0,10 mole) d'α-bromoacétophénone et de 11,4 g (0,15 mole) de thiourée dans 75 ml de méthanol. On évapore à sec sous pression réduite, et reprend le résidu d'évaporation par de l'eau. La solution résultante portée au reflux est traitée au noir CXA puis alcalinisée par de l'ammoniaque. Le précipité formé est isolé par filtration et purifié par recristallisation, avec traitement au noir CXA, de l'isopropanol pour donner 15 g (rendement : 85,15%) de produit attendu se présentant sous la forme d'aiguilles blanches insolubles dans l'eau. $F_{inst.}$ = 150°C.

PREPARATION III

On isole la base libre contenue dans le résidu d'évaporation du stade b) de la Préparation I. Par réaction de ladite base libre avec l'acide méthanesulfonique, l'acide fumarique et, respectivement, l'acide maléique, on obtient les méthanesulfonate, hémifumarate et, respectivement, hémimaléate de 2-(2-hydroxyéthylamino)-4-phénylthiazole (exemples 2-4).

On a résumé ci-après les résultats des essais qui ont été entrepris avec le CRL 41 108 (produit de l'exemple 1) selon l'invention. Dans ces essais, le CRL 41 108, en solution dans l'eau distillée (pH 3,5), a été administré par voie intrapéritonéale, sous un volume de 20 ml/kg chez la souris mâle, et, sous un volume de 5 ml/kg chez le rat mâle.

I - <u>TOXICITE</u>

L'étude de prétoxicité qui a été réalisée sur des lots de 3 animaux par dose étudiée, montre que chez la souris mâle par voie I.P.
- la DL-O (dose maximale non mortelle) est supérieure à 256 mg/kg,
- la DL-100 (dose minimale mortelle pour tous les animaux) est inférieure à 1024 mg/kg,
- la dose de 512 mg/kg provoque une sédation et une diminution de la réactivité au toucher et de la fréquence cardiaque, et entraîne la mort de 2 animaux sur 3, en 0,5 h,
- la DL-60 est de l'ordre de environ 500 mg/kg.

II- <u>COMPORTEMENT GLOBAL ET REACTIVITES</u>

Des lots de trois animaux sont observés avant, puis 0,25 h, 0,50 h, 1 h, 2 h, 3 h, et 24 h après l'administration du CRL 41 108. On constate ce qui suit :

1°) <u>chez la souris</u> :

- aux doses de 2 mg/kg et 8 mg/kg :
- aucune modification nette du comportement et des réactivités par rapport au lot témoin ne recevant que de l'eau distillée;
- à la dose de 32 mg/kg :
- une hypothermie (valeur maximale : -1,6°C) se manifestant pendant 1 h;
-aux doses de 64 mg/kg et 128 mg/kg :
- une sédation,
- une hypothermie (qui pour la dose de 128 mg/kg, a une valeur maximale de -5°C et se manifeste pendant 3 h),
- une diminution de la fréquence respiratoire,
- une diminution des réactivités, du tonus et de la force musculaire; et,

2°) <u>chez le rat</u> :

- aux doses de 1 mg/kg, 4 mg/kg et 16 mg/kg :
- aucune modification du comportement et des réactivités, par rapport aux animaux témoins,
- des variations de la température rectale et du diamètre pupillaire sensiblement analogues à celles des animaux témoins,
- à la dose de 64 mg/kg :
- une diminution de la fréquence cardiaque,
- une diminution des réactivités,
- une diminution de la réaction de peur,
- une diminution du tonus et de la force musculaire, et
- une hypothermie (valeur maximale -1,9°C) se manifestant pendant 1 h.

III - <u>INTERACTION AVEC L'APOMORPHINE</u>

1°) <u>chez la souris</u>

Des lots de 6 souris reçoivent le CRL 41 108 par voie I.P 0,5 h avant l'injection sous-cutanée de 1 mg/kg ou 16 mg/kg d'apomorphine. On observe que le CRL 41 108 induit une hypothermie aux doses de 8 mg/kg, 32 mg/kg et 128 mg/kg et aggrave, surtout aux doses de 32 mg/kg et 128 mg/kg, l'hypothermie provoquée par l' apomorphine, sans modifier l'attitude de verticalisation et les stéréotypies.

2°) <u>chez le rat</u>

Le CRL 41 108 est administré à des lots de 6 rats 0,5 h avant l'injection sous-cutanée de 0,5 mg/kg d'apomorphine. On observe que le CRL 41 108 ne modifie pas les stéréotypies induites par l'apomorphine.

IV - INTERACTION AVEC L'AMPHETAMINE

L'amphétamine (2 mg/kg) est injectée par voie intrapéritonéale à des lots de 6 rats 0,5 h après l'administration de CRL 41 108. On constate que le CRL 41 108, à la plus forte dose étudiée (64 mg/kg), provoque une diminution des stéréotypies amphétaminiques.

V - INTERACTION AVEC LA RESERPINE

Le CRL 41 108 est administré à des lots de 6 souris 0,5 h avant l'injection intrapéritonéale de 0,5 mg/kg d'oxotrémorine.

1°) Action sur la température

Le CRL 41 108, aux doses de 8 mg/kg, 32 mg/kg et 128 mg/kg est hypothermisant et aggrave l'hypothermie induite par l'oxotrémorine après un temps de latence de 1,5 h.

2°) Action sur les tremblements

Le CRL 41 108 ne modifie pas les tremblements dus à l'oxotrémorine.

3°) Action sur les symptômes cholinergiques périphériques.

On constate que le CRL 41 108 ne modifie pas les signes de stimulation cholinergique périphérique (salivation, lacrymation, défécation ) dûs à l'oxotrémorine.

VII - ACTION SUR LE TEST DES QUATRE PLAQUES, LA TRACTION ET L'ELECTROCHOC

Le test est pratiqué sur des lots de 10 souris 0,5 h après l'administration de CRL 41 108.
On observe que le CRL 41 108, aux doses de 32 mg/kg et 128 mg/kg, diminue le nombre de passages punis, qu'il ne provoque pas d'incoordination motrice et qu'il ne modifie pas les effets convulsivants et létaux de l'électrochoc.

VIII - ACTION SUR LA MOTILITE SPONTANEE

Une demi-heure après avoir reçu le CRL 41 108, les souris (6 par dose, 12 témoins) sont placées en actimètre où leur motilité est enregistrée pendant 30 minutes. On constate que le CRL 41 108, aux doses de 32 mg/kg et 128 mg/kg, diminue nettement l'activité motrice spontanée chez la souris.

IX - ACTION SUR L'AGRESSIVITE INTERGROUPES

Après avoir séjourné pendant 3 semaines dans chacune des moitiés d'une cage séparées par une cloison opaque, des groupes de 3 souris reçoivent le CRL 41 108. Une demi-heure plus tard, les deux groupes d'une même cage sont réunis par retrait de la cloison et on note le nombre de combats qui surviennent en 10 minutes.
On observe que, dès la dose de 0,5 mg/kg, le CRL 41 108 diminue de façon statistiquement significative le nombre de combats.

X - ACTION VIS-A-VIS DE QUELQUES COMPORTEMENTS PERTURBES PAR DIVERS AGENTS

1°) Motilité réduite par habituation à l'enceinte

Après 18 heures de séjour dans les actimètres, les souris (6 par dose, 12 témoins) reçoivent le CRL 41 108. Elles sont aussitôt replacées dans leurs enceintes respectives et, une demi-heure plus tard, on enregistre leur motilité pendant 30 minutes.
On note que le CRL 41 108 ne provoque pas de reprise nette de l'activité motrice chez la souris habituée à son enceinte.

2°) Motilité réduite par agression hypoxique

Une demi- heure après avoir reçu le CRL 41 108, les souris (10 par dose, 20 témoins) sont soumises à une anoxie hypobare aigüe [dépresssion de 600 mmHg (i.e. environ 8 x $10^4$ Pa) en 90 secondes, puis détente de 45 secondes], puis elles sont placées en actimètre où leur motilité est enregistrée pendant 10 minutes.
On constate que le CRL 41 108, à la plus forte dose étudiée (128 mg/kg), entraîne une faible détérioration de la récupération motrice chez les souris dont l'activité a été déprimée à la suite d'un bref séjour dans une enceinte à pression réduite.

3°) Anoxie asphyxique

Des lots de 10 souris reçoivent le CRL 41 108, 0,5 h avant l'administration intrapéritonéale de 32 mg/kg de triiodoéthylate de gallamine (substance curarisante de référence).

Le CRL 41 108 ne modifie pratiquement pas le délai d'apparition des convulsions et de la mort consécutives à une anoxie asphyxique provoquée par un curarisant.

XI - INTERACTION AVEC LE BARBITAL

Une demi-heure après l'administration de CRL 41 108 des lots de 10 souris reçoivent une injection intrapéritonéale de barbital (220 mg/kg).

On constate que, aux doses utilisées, le CRL 41 108 ne modifie pas la durée du sommeil barbiturique.

XII - ACTION SUR LE "DESESPOIR COMPORTEMENTAL"

Une demi-heure après avoir reçu le CRL 41 108, des lots de 6 souris sont placés dans un bécher rempli d'eau sur une hauteur de 6 cm. On note la durée totale d'immobilité entre la 2ème et la 6ème minute suivant l'immersion.

On observe que le CRL 41 108 diminue, aux doses de 32 mg/kg et surtout de 128 mg/kg, la durée de l'immobilité dite de "désespoir" de la souris placée en immersion forcée.

XIII - ACTION VIS-A-VIS DE LA SEROTONINE

On a voulu rechercher l'activité du CRL 41 108 vis-à-vis des tremblements induits chez la souris par la sérotonine (5-hydroxytryptamine ou 5-HT) associée à un inhibiteur de la monoamine-oxydase (en abrégé I.M.A.O), dans le cas d'espèce le nialimide.

Des lots de 20 souris mâles reçoivent le nialimide (20 mg/kg) par voie gastrique 18 h avant l'injection I.P. de (±) -5-HT (20 mg/kg), le CRL 41 108 étant injecté par voie I.P. 30 minutes avant le (±) -5-HT.

Immédiatement après l'injection de (±) -5-HT, les souris sont placées individuellement dans des boîtes transparentes en plastique ("plexiglas"). On note en tout ou rien les tremblements généralisés, les tremblements partiels, les secousses de la tête (en anglais : "head twitch") pendant 2,5 h. Les tremblements totaux regroupent les tremblements partiels et/ou généralisés.

On constate que, chez la souris prétraitée par un I.M.A.O., le CRL 41 108 n'entraîne pas l'apparition de tremblements généralisés après administration d'une dose liminaire de (±) -5-HT. A la plus forte dose utilisée (128 mg/kg), le CRL 41 108 semble diminuer les tremblements totaux et les secousses de la tête.

L'absence de potentialisation des effets comportementaux induits par le 5-HT chez la souris, rend improbable une participation sérotoninergique du CRL 41 108 dans les mécanismes responsables de son action anti-agressive dans le cadre du test de l'agressivité intergroupes sus-visé.

XIV - CONCLUSIONS

L'étude neuropsychopharmacologique met en évidence que le CRL 41 108 est un agent psychotrope sédatif (i) induisant une diminution de l'agressivité à une dose ne diminuant pas l'activité motrice spontanée, et (ii) diminuant la durée de désespoir comportemental de la souris.

En clinique, le CRL 41 108 s'est avéré être, en tant que sédatif, un excellent médicament calmant chez l'homme adulte. La posologie préconisée chez l'homme adulte est de 2 à 3 gélules par jour, renfermant chacune 25 mg de CRL 41 108.

**Revendications pour les états contractants: BE, CH, DE, FR, GB, IT, LI, LU, NL, SE**

1. Composition thérapeutique caractérisée en ce qu'elle renferme, en association avec un excipient physiologiquement acceptable, au moins un dérivé de 4-phénylthiazole choisi parmi le 2-(2-hydroxyéthylamino)-4-phénylthiazole et ses sels d'addition non-toxiques.

2. Composition suivant la revendication 1, caractérisée en ce que le dérivé de 4-phénylthiazole est le chlorhydrate de 2-(2-hydroxyéthylamino)-4-phénylthiazole.

3. Utilisation d'une substance appartenant à l'ensemble des dérivés de 4-phénylthiazole selon la revendication 1 et choisie parmi le 2-(2-hydroxyéthylamino)-4-phénylthiazole et ses sels d'addition non-toxiques, pour l'obtention d'un médicament sédatif.

4. Utilisation suivant la revendication 3, caractérisée en ce que ladite substance est le chlorhydrate de 2-(2-hydroxyéthylamino)-4-phénylthiazole.

5. Procédé de préparation d'un dérivé de 4-phénylthiazole choisi parmi l'ensemble constitué par

a) le 2-(2-hydroxyéthylamino)-4-phénylthiazole, et

b) ses sels d'addition, ledit procédé étant caractérisé en ce que
1) on fait réagir le 2-amino-4-phénylthiazole de formule

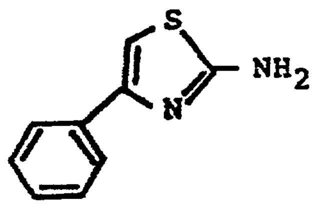

(II)

avec un halogénure d'un monoester d'alkyle inférieur de l'acide oxalique de formule
HalCO–COOAlk (III)
où Hal représente F, Cl ou Br, et Alk représente un groupe alkyle inférieur en $C_1$–$C_2$, pour obtenir un dérivé 2-alkyloxalylamido-4-phénylthiazole de formule

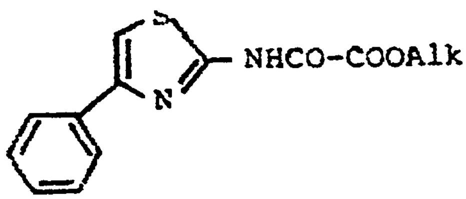

(IV)

où Alk ets défini comme indiqué ci-dessus, puis
2) on réduit ledit composé de formule IV, ainsi obtenu, au moyen de $AlLiH_4$.

6. Procédé selon la revendication 5, caractérisé en ce que la réaction du stade 1) est mise en œuvre dans un solvant organique anhydre, pendant au moins 0,25 h, à raison de plus d'une mole de III pour une mole de II, en présence d'un moyen accepteur de proton.

7. Procédé selon la revendication 5, caractérisé en ce que la réaction de réduction du stade 2) est réalisée dans un solvant organique anhydre, à raison de plus de deux moles de $AlLiH_4$ pour une mole de composé IV.

8. Procédé selon la revendication 6, caractérisé en ce que l'on utilise 1,1 mole de III pour 1 mole de II, et en ce que le moyen accepteur de proton est choisi parmi la pyridine et la picoline.

9. Procédé selon revendication 7, caractérisé en ce que l'on utilise 3 moles de $AlLiH_4$ pour 1 mole de composé IV, et en ce que le solvant organique anhydre est le tétrahydrofuranne.

**Revendications pour les états contractants: AT, ES, GR**

1. Procédé de préparation d'un dérivé de 4-phénylthiazole choisi parmi l'ensemble constitué par
a) le 2-(2-hydroxyéthylamino)-4-phénylthiazole, et
b) ses sels d'addition,
ledit procédé étant caractérisé en ce que
1) on fait réagir le 2-amino-4-phénylthiazole de formule

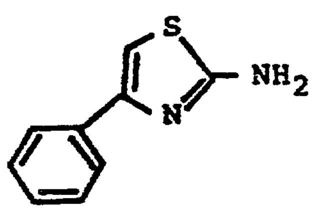

(II)

avec un halogénure d'un monoester d'alkyle inférieur de l'acide oxalique de formule
HalCO–COOAlk (III)
où Hal représente F, Cl ou Br, et Alk représente un groupe alkyle inférieur en $C_1$–$C_2$, pour obtenir un dérivé 2-alkyloxalylamido-4-phénylthiazole de formule

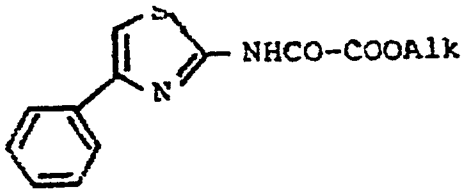

(IV)

où Alk est défini comme indiqué ci-dessus, puis

2) on réduit ledit composé de formule IV, ainsi obtenu, au moyen de $AlLiH_4$.

2. Procédé selon la revendication 1, caractérisé en ce que la réaction du stade 1) est mise en œuvre dans un solvant organique anhydre, pendant au moins 0,25 h, à raison de plus d'une mole de III pour une mole de II, en présence d'un moyen accepteur de proton.

3. Procédé selon la revendication 1, caractérisé en ce que la réaction de réduction du stade 2) est réalisée dans un solvant organique anhydre, à raison de plus de deux moles de $AlLiH_4$ pour une mole de composé IV.

4. Procédé selon la revendication 2, caractérisé en ce que l'on utilise 1,1 mole de III pour 1 mole de II, et en ce que le moyen accepteur de proton est choisi parmi la pyridine et la picoline.

5. Procédé selon la revendication 3, caractérisé en ce que l'on utilise 3 moles de $AlLiH_4$ pour 1 mole de composé IV, et en ce que le solvant organique anhydre est le tétrahydrofuranne.

**Claims for the Contracting States: AT, ES, GR**

1. A method for the preparation of a 4-phenylthiazole derivative selected from the group comprising:

a) 2-(2-hydroxyethylamino)-4-phenylthiazole and

b) its addition salts,

the said method comprising:

1) reacting 2-amino-4-phenylthiazole of the formula:

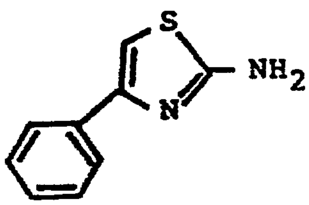

(II)

with a halide of a lower alkyl monoester of oxalic acid of the formula:

HalCO–COOAlk (III)

in which Hal represents F, Cl or Br, and Alk represents a $C_1$–$C_2$ lower alkyl group, to give a 2-alkyloxalylamido-4-phenylthiazole derivative of the formula:

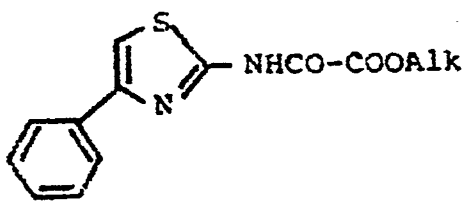

(IV)

in which Alk is defined as indicated above, and then

2) reducing the said compound of the formula IV, thus obtained with $AlLiH_4$.

2. The method as claimed in claim 1, wherein the reaction of stage 1) is carried out in an anhydrous organic solvent, for at least 0.25 h, at a rate of more than one mol of III per mol of II, in the presence of a proton acceptor.

3. The method as claimed in claim 1, wherein the reduction reaction of stage 2) is carried out in an anhydrous organic solvent at a rate of more than two mol of $AlLiH_4$ per mol of compound IV.

4. The method as claimed in claim 2, wherein 1.1 mol of III are used per mol of II and wherein the proton acceptor is selected from pyridine and picoline.

5. The method as claimed in claim 3, wherein 3 mol of $AlLiH_4$ are used per mol of compound IV and wherein the anhydrous organic solvent is tetrahydrofuran.

**Claims for the Contracting States: BE, CH, DE, FR, GB, IT, LI, LU, NL, SE**

1. A therapeutic composition containing, in association with a physiologically acceptable excipient, at least one 4-phenylthiazole derivative selected from the group comprising 2-(2-hydroxyethylamino)-4-phenylthiazole and its non-toxic addition salts.

2. A composition as claimed in claim 1, wherein the 4-phenylthiazole derivative is 2-(2-hydroxyethylamino)-4-phenylthiazole hydrochloride.

3. Use of a substance belonging to the series of 4-phenylthiazole derivatives as claimed in claim 1, and selected from the group comprising 2-(2-hydroxyethylamino)-4-phenylthiazole and its non-toxic addition salts, for the preparation of a sedative drug.

4. Use as claimed in claim 3, wherein the said substance is 2-(2-hydroxyethylamino)-4-phenylthiazole hydrochloride.

5. A method for the preparation of a 4-phenylthiazole derivative selected from the group comprising:

a) 2-(2-hydroxyethylamino)-4-phenylthiazole and

b) its addition salts,

the said method comprising:

1) reacting 2-amino-4-phenylthiazole of the formula:

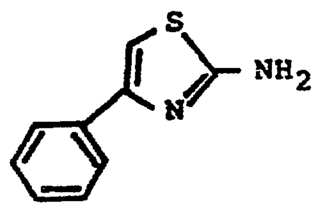

(II)

with a halide of a lower alkyl monoester of oxalic acid of the formula:

HAlCO–COOAlk (III)

in which Hal represents F, Cl or Br, and Alk represents a $C_1$–$C_2$ lower alkyl group, to give a 2-alkyloxalylamido-4-phenylthiazole derivative of the formula:

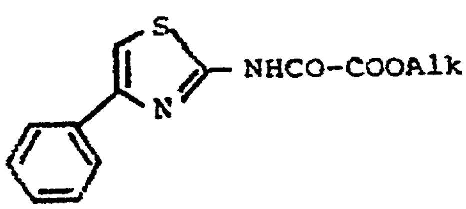

(IV)

in which Alk is defined as indicated above, and then

2) reducing the said compound of the formula IV, thus obtained with $AlLiH_4$.

6. The method as claimed in claim 5, wherein the reaction of stage 1) is carried out in an anhydrous organic solvent, for at least 0.25 h, at a rate of more than one mole of III per mol of II, in the presence of a proton acceptor.

7. The method as claimed in claim 5, wherein the reduction reaction of stage 2) is carried out in an anhydrous organic solvent at a rate of more than two mol of $AlLiH_4$ per mol of compound IV.

8. The method as claimed in claim 6, wherein 1.1 mol of III are used per mol of II and wherein the proton acceptor is selected from pyridine and picoline.

9. The method as claimed in claim 7, wherein 3 mol of $AlLiH_4$ are used per mol of compound IV and wherein the anhydrous organic solvent is tetrahydrofuran.

**Patentansprüche für die Vertragsstaaten: AT, ES, GR**

1. Verfahren zur Herstellung eines 4-Phenylthiazol-Derivats, ausgewählt aus der Gruppe, die gebildet ist von

a) 2-(2-Hydroxyethylamino)-4-phenylthiazol und

b) dessen Additionssalzen,

dadurch gekennzeichnet, daß

1) 2-Amino-4-phenylthiazol der Formel

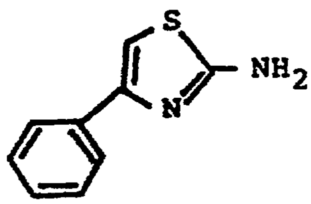

(II)

umgesetzt wird mit einem Halogenid eines niederen Monoalkylesters der Oxalsäure der Formel
HalCO–COOAlk (III)
worin Hal F, Cl oder Br bedeutet und Alk eine niedere Alkylgruppe mit $C_1$–$C_2$ ist, um ein 2-Alkyloxalylamido-4-phenylthiazol der Formel

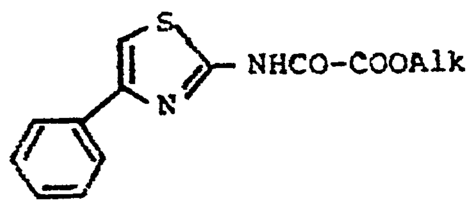

(IV)

zu erhalten, worin Alk die oben angegebene Bedeutung hat, und dann
2) die Verbindung nach Formel IV mit $AlLiH_4$ reduziert wird.

2. Verfahren nach Anspruch 1, dadurch gekennzeichnet, daß die Reaktion der Stufe 1) mindestens 0,25 h lang in einem wasserfreien organischen Lösungsmittel in einem Verhältnis von mehr als einem Mol III auf ein Mol II in Gegenwart eines Protonenakzeptors erfolgt.

3. Verfahren nach Anspruch 1, dadurch gekennzeichnet, daß die Reduktionsreaktion der Stufe 2) in einem wasserfreien organischen Lösungsmittel im Verhältnis von mehr als zwei Mol $AlLiH_4$ auf ein Mol Verbindung IV erfolgt.

4. Verfahren nach Anspruch 2, dadurch gekennzeichnet, daß 1,1 Mol der Verbindung III auf 1 Mol der Verbindung II verwendet wird, und daß als Protonenakzeptor Pyridin oder Picolin dienen.

5. Verfahren nach Anspruch 3, dadurch gekennzeichnet, daß 3 Mol $AlLiH_4$ auf 1 Mol der Verbindung IV verwendet werden, und daß Tetrahydrofuran als wasserfreies organisches Lösungsmittel dient.

**Patentansprüche für die Vertragsstaaten: BE, CH, DE, FR, GB, IT, LI, LU, NL, SE**

1. Therapeutische Zusammensetzung, dadurch gekennzeichnet, daß sie in Verbindung mit einer physiologisch annehmbaren Grundmasse mindestens ein Derivat von 4-Phenylthiazol enthält, da ausgewählt ist unter 2-(2-Hydroxyethylamino)-e-phenylthiazo und dessen ungiftigen Aditionssalzen.

2. Zusammensetzung nach Anspruch 1, dadurch gekennzeichnet, daß das 4-Phenylthiazol-Derivat 2-(2-Hydroxyethylamino)-4-phenylthiazol-chlorhydrat ist.

3. Verwendung einer zur Gruppe der 4-Phenylthiazol-Derivate gemäß Anspruch 1 gehörigen und zwischen 2-(2-Hydroxyethylamino)-4-phenylthiazol und dessen ungiftigen Additionssalzen ausgewählten Substanz zur Gewinnung eines sedierend wirkenden Arzneimittels.

4. Verwendung nach Anspruch 3, dadurch gekennzeichnet, daß die genannte Substanz 2-(2-Hydroxyethylamino)-4-phenylthiazol-chlorhydrat ist.

5. Verfahren zur Herstellung eines 4-Phenylthiazol-Derivats, ausgewählt aus der Gruppe, die gebildet ist von:
a) 2-(2-Hydroxyethylamino)-4-phenylthiazol und
b) dessen Additionssalzen,
dadurch gekennzeichnet, daß
1) 2-Amino-4-phenylthiazol der Formel

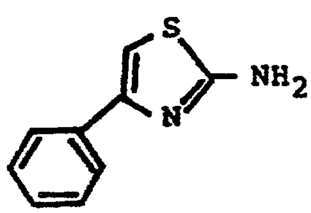

(II)

umgesetzt wird mit einem Halogenid eines niederen Monoalkylesters der Oxalsäure der Formel

HalCO–COOAlk (III)
worin Hal F, Cl oder Br bedeutet und Alk eine niedere Alkylgruppe mit $C_1$–$C_2$ ist, um ein 2-Alkyloxalylamido-4-phenylthiazol der Formel

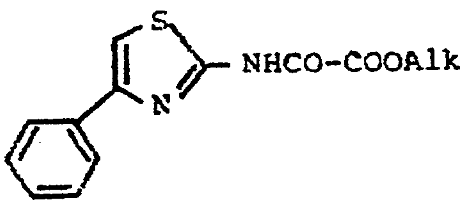

(IV)

zu erhalten, worin Alk die oben angegebene Bedeutung hat, und dann
2) die Verbindung nach Formel IV mit $AlLiH_4$ reduziert wird.

6. Verfahren nach Anspruch 5, dadurch gekennzeichnet, daß die Reaktion der Stufe 1) mindestens 0,25 h lang in einem wasserfreien organischen Lösungsmittel in einem Verhältnis von mehr als einem Mol III auf ein Mol II in Gegenwart eines Protonenakzeptors erfolgt.

7. Verfahren nach Anspruch 5, dadurch gekennzeichnet, daß die Reduktionsreaktion der Stufe 2) in einem wasserfreien organischen Lösungsmittel im Verhältnis von mehr als zwei Mol $AlLiH_4$ auf ein Mol Verbindung IV erfolgt.

8. Verfahren nach Anspruch 6, dadurch gekennzeichnet, daß 1,1 Mol der Verbindung III auf 1 Mol der Verbindung II verwendet wird, und daß als Protonenakzeptor Pyridin oder Picolin dienen.

9. Verfahren nach Anspruch 7, dadurch gekennzeichnet, daß 3 Mol $AlLiH_4$ auf 1 Mol der Verbindung IV verwendet werden, und daß Tetrahydrofuran als wasserfreies organisches Lösungsmittel dient.